**Europäisches Patentamt**

**European Patent Office**

**Office européen des brevets**

(19)

(11) Publication number : **0 440 398 B1**

# (12) EUROPEAN PATENT SPECIFICATION

(45) Date of publication of patent specification :
29.12.93 Bulletin 93/52

(51) Int. Cl.⁵ : **A61K 7/48**

(21) Application number : **91300627.6**

(22) Date of filing : **28.01.91**

(54) **Skin care compositions.**

The file contains technical information
submitted after the application was filed and
not included in this specification

(30) Priority : **29.01.90 US 471760**

(43) Date of publication of application :
**07.08.91 Bulletin 91/32**

(45) Publication of the grant of the patent :
**29.12.93 Bulletin 93/52**

(84) Designated Contracting States :
**DE ES FR GB IT**

(56) References cited :
**EP-A- 0 330 496**
**EP-A- 0 343 444**
**AT-B- 364 711**
**FR-A- 2 247 203**

(73) Proprietor : **JOHNSON & JOHNSON**
**CONSUMER PRODUCTS, INC.**
**501 George Street**
**New Brunswick New Jersey 08903 (US)**

(72) Inventor : **Clum, Charles E.**
**60 Brophy Drive**
**Ewing, New Jersey 08638 (US)**
Inventor : **Wang, Jonas, C.T.**
**23 Ellsworth Drive**
**Robbinsville, New Jersey 08691 (US)**

(74) Representative : **Mercer, Christopher Paul et al**
**Carpmaels & Ransford 43, Bloomsbury Square**
**London WC1A 2RA (GB)**

## Description

FIELD OF THE INVENTION

This invention relates to skin care compositions containing retinoids which generally improve the quality of the skin, particularly human facial skin.

BACKGROUND OF THE INVENTION

Skin care compositions containing retinoids have become a focus of great interest in recent years. A number of products have appeared in the market place with therapeutic claims against photoaging and sun-damaged skin.

Caucasians who have had a good deal of sun exposure in childhood will show the following gross cutaneous alterations in later adult life: wrinkling, leatheriness, yellowing, looseness, roughness, dryness, mottling (hyperpigmentation) and various premalignant growths (often subclinical). These changes are most prominent in light-skinned persons who burn easily and tan poorly. These cumulative effects of sunlight are often referred to as "photoaging". Although the anatomical degradation of the skin is most advanced in the elderly, the destructive effects of excessive sun exposure are already evident by the second decade. Serious microscopic alterations of the epidermis and dermis occur decades before these become clinically visible. Wrinkling, yellowing, leatheriness and loss of elasticity are very late changes.

United States patent No. 4,603,146 entitled "Composition and Method for Improving the Quality of Human Skin and Skin Aging Retardant" discloses methods for treating sun-damaged human skin topically with Vitamin A acid, which is a retinoid, in an emollient vehicle in such amounts as to be essentially non-irritating to the skin. This treatment causes the skin, particularly human facial skin, to substantially regain and maintain its firmness, turgor and elasticity by retarding and reversing the skin's loss of collagen fibers, abnormal changes in elastic fibers, deterioration of small blood vessels, epidermal atrophy and formation of abnormal epithelial growths.

United States patent No. 4,877,805 entitled "Methods for Treatment of Sun-damaged Human Skin with Retinoids" covers a method for retarding and reversing the loss of collagen fibers, abnormal changes in elastic fibers, deterioration of small blood vessels and formation of abnormal epithelial growths in sun-damaged human skin comprising applying topically a composition comprising a retinoid in an emollient vehicle in a program of maintenance therapy. A number of retinoids are disclosed as useful in this invention.

A number of skin care products have appeared in the marketplace with claims related to the repair of wrinkled and/or photoaged or sun-damaged skin and are alleged to contain retinoids such as retinol, retinyl palmitate and retinyl acetate. All of these products appear to be oil-in-water emulsion systems, i.e. systems characterized by an oil phase dispersed in a water phase. Problems occur in utilizing retinoids in such a system since they are unstable therein and chemically degrade and are, therefore, unavailable over time for their alleged utility.

SUMMARY OF THE INVENTION

It is an object of the present invention to provide improved skin care compositions.

It is another object of the present invention to provide improved skin care compositions for the treatment of sun-damaged skin.

It is a further object of this invention to provide improved skin care compositions that contain retinoids and possess good physical and chemical stability.

Other objects of this invention will be set forth in or be apparent from the following detailed description of the invention.

The foregoing objects and other features and advantages of the present invention are achieved by skin care compositions comprising at least one active ingredient in an emulsion system. More specifically, the present invention relates to skin care compositions comprising a retinoid as the active ingredient in a specific emulsion system.

DETAILED DESCRIPTION OF THE INVENTION

In general, this invention relates to a skin care composition comprising a stable water-in-oil emulsion base including an antioxidant system, a chelating agent and at least one retinoid compound.

An emulsion is usually thought of as an intimate mixture of two immiscible liquids which exhibits an acceptable shelf life at or about room temperature. When two immiscible liquids are mechanically agitated, both phases initially tend to form droplets. When the agitation ceases, the droplets quickly coalesce, and the two liquids separate. The lifetime of the droplets is materially increased if a compound referred to as an emulsifier is added to the immiscible liquids. Usually only one phase persists in droplet form for a prolonged period of time, and this is referred to as the internal phase which is surrounded by an external phase.

Most commercial skin care compositions containing retinoids are oil-in-water emulsion systems, and in such systems the retinoid compounds, in particular, retinol and the retinyl esters tend to be unstable, i.e. they degrade and are, therefore, not available to perform in their desired manner. It is believed that this degradation occurs as a result of the solubility and rapid diffusion of oxygen through the external water phase to the internal oil phase containing the retinoid, and degradation of the retinoid then occurs. Since the solubility and diffusion of oxygen is greater in a water phase than an oil phase, an oil-in-water system is more prone to such degradation.

On the other hand, water-in-oil emulsions are difficult to process because the continuous or external phase is oil and stability problems resulting in separate phases occur more frequently in such systems than in oil-in-water emulsion systems. Also in the past, water-in-oil emulsions have been perceived to be less cosmetically appealing and greasier due to the continuous phase being an oil phase.

The present invention has overcome these difficulties and provides a water-in-oil emulsion composition containing at least one retinoid compound wherein the stability of both the emulsion and the active ingredients is excellent.

The skin care compositions of the present invention comprise a water-in-oil emulsion base and an antioxidant system, a chelating agent and at least one retinoid compound.

The antioxidant system useful in the present invention comprises at least one water-soluble antioxidant and at least one oil-soluble antioxidant. The water-soluble antioxidant protects the retinoid compounds from endogeneous oxidation and the oil-soluble antioxidant protects the retinoid compounds from exogeneous oxidation. Both antioxidants are necessary in the compositions of the present invention.

The water-soluble antioxidants which are useful in the compositions of the present invention include ascorbic acid, sodium sulfite, sodium metabisulfite, sodium bisulfite, sodium thiosulfite, sodium formaldehyde sulfoxylate, isoascorbic acid, thioglycerol, thiosorbitol, thiourea, thioglycolic acid, cysteine hydrochloride, 1,4-diazobicyclo-(2,2,2)-octane and mixtures thereof as well as any other known water-soluble antioxidant compatible with the other components of the compositions.

The oil-soluble antioxidants which are useful in the compositions of the present invention include butylated hydroxytoluene (BHT), ascorbyl palmitate, butylated hydroxyanisole, $\alpha$-tocopherol, phenyl-$\alpha$-naphthylamine, and mixtures thereof as well as any other known oil-soluble antioxidant compatible with the other components of the compositions.

Certain antioxidants function as both water-soluble and oil-soluble antioxidants and formulations containing such antioxidants are within the scope of this invention. Hydroquinone, propyl gallate, nordihydroguiaretic acid and mixtures thereof are examples of such antioxidants.

The antioxidants should be utilized in an effective amount and may range in total from 0.0001 to 5.0% based on the total composition, preferably from 0.01 to 1.0%. The amount of antioxidants utilized in the compositions of the present invention is dependent in part on the specific antioxidants selected, the amount of and specific retinoid being protected and the processing conditions.

Retinoid compounds are known to be very sensitive to metal ions and in particular to bi- and tri-valent cations and degrade rapidly in their presence. For that reason, it is necessary to add a chelating agent to the compositions of the present invention. The chelating agent forms a complex with the metal ions thereby inactivating them and preventing them from affecting the retinoid compounds. Any suitable chelating agent which is compatible with the components of the compositions of the present invention may be utilized such as ethylenediamine tetracetic acid (EDTA) and derivatives and salts thereof, dihydroxyethyl glycine, citric acid, tartaric acid, and mixtures thereof. The chelating agents should be utilized in an effective amount and may range from 0.01 to 2.0% based on the total compositions, preferably from 0.05 to 1.0%.

The retinoid compounds which are useful in the compositions of the present invention consist of Vitamin A alcohol (retinol) and its derivatives such as Vitamin A aldehyde (retinal) and Vitamin A esters (retinyl acetate and retinyl palmitate).

The retinoids that are utilized in the compositions of the present invention are present in a therapeutically effective amount that may range from 0.0001 to 5.0% by weight of the total compositions, preferably from 0.001 to 1.0%.

The skin care compositions of the present invention comprising a water-in-oil emulsion can be in the format of cream or lotion formulations, as desired, by varying the oil and water phases of the emulsion.

Minerals oils, animal oils, vegetable oils and silicones have all been used in cosmetic creams and lotions of the emulsion type. In addition to such oils, other emollients and surface active agents have been incorporated in the emulsions, including stearates, such as potassium stearate, glycol stearate, sodium stearate, polyethylene glycol (40) stearate and glyceryl stearate; laurates, such as sodium laurate and potassium laurate; alcohols, such as cetyl alcohol and lanolin alcohol; triethanolamine; myristates, such as isopropyl myristate, sodium myristate and potassium myristate; cetyl palmitate; cholesterol; stearic acid; sorbitan sesquioleate; propylene glycol; glycerine, sorbitol and the like. Thickeners such as natural gums and synthetic polymers, as well as preservatives such as methylparaben, and propylparaben, coloring agents and fragrances also are commonly included in such compositions. Other active ingredients such as sunscreen materials and antimicrobial materials may be utilized in the compositions of the present invention provided that they are physically and chemically compatible with the other components of the compositions.

The essence of the present invention is not within the specific composition per se of the cream or lotion formulation, and any of the many formulations or compositions of the cream or lotion type currently utilized in skin care preparations can be employed provided that it is in a water-in-oil emulsion base with the appropriate antioxidant system and chelating agent and is chemically compatible with the retinoid compounds. The ratio of the oil phase of the emulsion to the water phase can be from 5:95 to 99:1. The actual ratio of the two phases will depend on the desired final product.

The compositions of the present invention can be prepared by well-known mixing or blending procedures. Each phase of the emulsion is preferable separately prepared with all of the components contained in the appropriate phase except it is usually preferred to omit the retinoid compound. The emulsion is then formed normally by adding the water phase to the oil phase with agitation, and often the emulsion is cooled down when the retinoid compound is added.

Specific embodiments of the skin care compositions prepared in accordance with the present invention are illustrated by the following representative examples.

EXAMPLE I

A water-in-oil cream composition is prepared according to the following procedure. In a suitable sized glass beaker 250 g mineral oil, 2 g propylparaben, 60 g Elfacos C-26, 50 g Elfacos E200, 30 g Elfacos ST-9, 10 g stearoxytrimethylsilane, 10 g dimethicone (50cstk), and 0.5 g butylated hydroxytoluene (BHT) are heated to 80°C and mixed. In a separate glass container approximately 520 g deionized water, 50 g sorbitol solution 70%, 3 g methylparaben, 1 g disodium edetate and 1 g ascorbic acid are mixed, adjusted to pH 4.7 with dilute sodium hydroxide Q.S., heated to 80°C. and then added to the first mixture with agitation to form a water-in-oil emulsion. The emulsion is homogenized and cooled to about 50°C. with mixing. 1 g Dowicil 200 preservative and 2.5 g fragrance followed by 1.65 g of retinol are added. Deionized water is added to return the batch weight to 1000 g and the batch is mixed until uniform and the temperature is below 40°C. The finished batch is filled into suitable containers and has the following formulation:

| Ingredient | % w/w |
|---|---|
| mineral oil | 25.000 |
| hydroxyoctacosanyl hydroxystearate (Elfacos C26) | 6.000 |
| sorbitol solution | 5.000 |
| methoxy PEG-22/dodecyl glycol copolymer (Elfacos E200) | 5.000 |
| PEG-45/dodecyl glycol copolymer (Elfacos ST9) | 3.000 |
| stearoxytrimethylsilane | 1.000 |
| dimethicone (50 cstk) | 1.000 |
| Vitamin A alcohol (retinol) | 0.165 |
| methylparaben | 0.300 |
| fragrance | 0.250 |
| propylparaben | 0.200 |
| Quaternium 15 (Dowicil 200) | 0.100 |
| disodium edetate | 0.100 |
| ascorbic acid | 0.100 |
| butylated hydroxytoluene | 0.050 |
| 50% aqueous NaOH | q.s. to pH 4.7 |
| deionized water | q.s. to 100.000 |

The above water-in-oil emulsion composition is a smooth off-white cream having a room temperature shelf life stability of the retinol of over two years.

EXAMPLE II (comparative example)

An oil-in-water cream composition is prepared in a suitable size glass beaker by first dispersing 40 g propylene glycol in 815.6 g deionized water and thereafter dispersing 5 g of Carbomer 934, 1 g ascorbic acid, 1 g disodium edetate and 1.5 g methylparaben and heating the resultant mix to 80°C. In a separate glass beaker are charged 15 g myristyl myristate, 12.5 g oleic acid, 12.5 g glyceryl stearate, 12.5 g stearic acid, 10 g isopropyl palmitate, 10 g stearoxytrimethylsilane, 5 g synthetic beeswax, 5 g stearyl alcohol, 5 g cetyl alcohol, 12 g Polysorbate 61, 10 g dimethicone, 1 g propylparaben, 0.2 g butylated hydroxytoluene, 0.5 g butylparaben and 8 g sorbitan stearate. The mixture is melted and the temperature is adjusted to 80°C. The oil phase is added to the aqueous portion with agitation to form an oil-in-water emulsion. 50% aqueous sodium hydroxide solution is added q.s. to pH 4.7 and the emulsion is cooled to 50°C. and 3 g benzyl alcohol and 2.5 g fragrance are added. 3.75 g of retinol are then added and deionized water is added to return the batch weight to 1000 g and the batch is mixed until uniform and the temperature is below 40°C. The finished batch is filled into suitable containers and has the following formulation:

| Ingredient | % w/w |
|---|---|
| propylene glycol | 4.000 |
| myristyl myristate | 1.500 |
| oleic acid | 1.250 |
| glyceryl stearate | 1.250 |
| stearic acid | 1.250 |
| Polysorbate 61 | 1.200 |
| stearoxytrimethylsilane | 1.000 |
| dimethicone (50 cstk) | 1.000 |
| isopropyl palmitate | 1.000 |
| sorbitan stearate | 0.800 |
| cetyl alcohol | 0.500 |
| stearyl alcohol | 0.500 |
| Carbomer 934 | 0.500 |
| synthetic beeswax | 0.500 |
| Vitamin A alcohol (retinol) | 0.375 |
| methylparaben | 0.300 |
| benzyl alcohol | 0.300 |
| fragrance | 0.250 |
| propylparaben | 0.200 |
| ascorbic acid | 0.100 |
| disodium edetate | 0.100 |
| butylated hydroxytoluene | 0.050 |
| butylparaben | 0.050 |
| 50% aqueous NaOH | q.s. to pH 4.7 |
| deionized water | q.s. to 100.000 |

The above oil-in-water emulsion composition is a smooth off-white cream having a room temperature shelf life stability of the retinol of less than three months and would not be a desirable commercial product.

EXAMPLE III

A water-in-oil cream composition is prepared in accordance with the procedure of Example I and consists of the following ingredients:

6

| Ingredient | % w/w |
|---|---|
| mineral oil | 25.000 |
| hydroxyoctacosanyl hydroxystearate (Elfacos C26) | 6.000 |
| sorbitol solution | 5.000 |
| methoxy PEG-22/dodecyl glycol copolymer (Elfacos E200) | 5.000 |
| PEG-45/dodecyl glycol copolymer (Elfacos ST9) | ·3.000 |
| stearoxytrimethylsilane | 1.000 |
| dimethicone (50 cstk) | 1.000 |
| Vitamin A alcohol (retinol) | 0.345 |
| methylparaben | 0.300 |
| fragrance | 0.250 |
| propylparaben | 0.200 |
| Quaternium 15 (Dowicil 200) | 0.100 |
| disodium edetate | 0.100 |
| ascorbic acid | 0.100 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE IV

A water-in-oil cream composition is prepared in accordance with the procedure of Example I and consists of the following ingredients:

| Ingredient | % w/w |
|---|---|
| mineral oil | 25.000 |
| hydroxyoctacosanyl hydroxystearate (Elfacos C26) | 6.000 |
| sorbitol solution | 5.000 |

| | |
|---|---|
| methoxy PEG-22/dodecyl glycol copolymer (Elfacos E200) | 5.000 |
| PEG-45/dodecyl glycol copolymer (Elfacos ST9) | 3.000 |
| stearoxytrimethylsilane | 1.000 |
| dimethicone (50 cstk) | 1.000 |
| Vitamin A alcohol (retinol) | 1.000 |
| methylparaben | 0.300 |
| fragrance | 0.250 |
| propylparaben, NF | 0.200 |
| Quaternium 15 (Dowicil 200) | 0.100 |
| disodium edetate | 0.100 |
| ascorbic acid | 0.100 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE V

A water-in-oil cream composition is prepared in accordance with the procedure of Example I and consists of the following ingredients:

| Ingredient | % w/w |
|---|---|
| mineral oil | 25.000 |
| hydroxyoctacosanyl hydroxystearate (Elfacos C26) | 5.000 |
| sorbitol solution | 5.000 |
| methoxy PEG-22/dodecyl glycol copolymer (Elfacos E200) | 5.000 |

8

| | |
|---|---|
| PEG-45/dodecyl glycol copolymer (Elfacos ST9) | 3.000 |
| stearoxytrimethylsilane | 1.000 |
| dimethicone (50 cstk) | 1.000 |
| Vitamin A alcohol (retinol) | 0.100 |
| methylparaben | 0.300 |
| fragrance | 0.250 |
| propylparaben | 0.200 |
| Quaternium 15 (Dowicil 200) | 0.100 |
| disodium edetate | 0.100 |
| sodium bisulfite | 0.100 |
| butylated hydroxytoluene | 0.050 |
| 50%· aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE VI

A water-in-oil cream composition is prepared in accordance with the procedure of Example I with the addition of 10 g trihydroxystearin as a thickener to the phase containing the mineral oil and consists of the following ingredients:

| Ingredient | % w/w |
|---|---|
| mineral oil | 25.000 |
| hydroxyoctacosanyl hydroxystearate (Elfacos C26) | 5.000 |
| sorbitol solution | 5.000 |
| methoxy PEG-22/dodecyl glycol copolymer (Elfacos E200) | 5.000 |

| | |
|---|---|
| PEG-45/dodecyl glycol copolymer (Elfacos ST9) | 3.000 |
| stearoxytrimethylsilane | 1.000 |
| dimethicone (50 cstk) | 1.000 |
| trihydroxystearin | 1.000 |
| Vitamin A alcohol (retinol) | 0.100 |
| methylparaben | 0.300 |
| fragrance | 0.250 |
| propylparaben | 0.200 |
| Quaternium 15 (Dowicil 200) | 0.100 |
| disodium edetate | 0.100 |
| ascorbic acid | 0.100 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE VII

A water-in-oil cream composition is prepared in accordance with the procedure of Example I with 30 g C18-36 acid triglyceride replacing Elfacos C-26 and consists of the following ingredients:

| Ingredient | % w/w |
|---|---|
| mineral oil | 25.000 |
| C18-36 acid triglyceride | 5.000 |
| sorbitol solution | 5.000 |
| methoxy PEG-22/dodecyl glycol copolymer (Elfacos E200) | 5.000 |

10

| | |
|---|---|
| PEG-45/dodecyl glycol | 3.000 |
| copolymer (Elfacos ST9) | |
| stearoxytrimethylsilane | 1.000 |
| dimethicone (50 cstk) | 1.000 |
| Vitamin A alcohol (retinol) | 0.100 |
| methylparaben | 0.300 |
| fragrance | 0.250 |
| propylparaben | 0.200 |
| Quaternium 15 (Dowicil 200) | 0.100 |
| disodium edetate | 0.100 |
| ascorbic acid | 0.100 |
| butylated hydroxytoluene | 0.050 |
| 50%. aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE VIII

A water-in-oil cream composition is prepared in accordance with the procedure of Example I with 70 g polyethylene replacing Elfacos C-26 and consists of the following ingredients:

| Ingredient | % w/w |
|---|---|
| mineral oil | 25.000 |
| polyethylene | 7.000 |
| sorbitol solution | 5.000 |
| methoxy PEG-22/dodecyl glycol | 5.000 |
| copolymer (Elfacos E200) | |
| PEG-45/dodecyl glycol | 3.000 |
| copolymer (Elfacos ST9) | |

| | |
|---|---|
| stearoxytrimethylsilane | 1.000 |
| dimethicone (50 cstk) | 1.000 |
| Vitamin A alcohol (retinol) | 0.100 |
| methylparaben | 0.300 |
| fragrance | 0.250 |
| propylparaben | 0.200 |
| Quaternium 15 (Dowicil 200) | 0.100 |
| disodium edetate | 0.100 |
| ascorbic acid | 0.100 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE IX

A water-in-oil cream composition is prepared in accordance with the procedure of Example I with the addition of 20 g silicon dioxide to the phase containing the mineral oil and consists of the following ingredients:

| Ingredient | % w/w |
|---|---|
| mineral oil | 25.000 |
| hydroxyoctacosanyl hydroxystearate (Elfacos C26) | 5.000 |
| sorbitol solution | 5.000 |
| methoxy PEG-22/dodecyl glycol copolymer (Elfacos E200) | 5.000 |
| PEG-45/dodecyl glycol copolymer (Elfacos ST9) | 3.000 |
| silicon dioxide | 2.000 |

| | |
|---|---|
| stearoxytrimethylsilane | 1.000 |
| dimethicone (50 cstk) | 1.000 |
| Vitamin A alcohol (retinol) | 0.345 |
| methylparaben | 0.300 |
| fragrance | 0.250 |
| propylparaben | 0.200 |
| Quaternium 15 (Dowicil 200) | 0.100 |
| disodium edetate | 0.100 |
| ascorbic acid | 0.100 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE X

A water-in-oil cream composition is prepared in accordance with the procedure of Example I with 1 g hydroquinone replacing the ascorbic acid and consists of the following ingredients:

| Ingredient | % w/w |
|---|---|
| mineral oil | 25.000 |
| hydroxyoctacosanyl hydroxystearate (Elfacos C26) | 5.000 |
| sorbitol solution | 5.000 |
| methoxy PEG-22/dodecyl glycol copolymer (Elfacos E200) | 5.000 |
| PEG-45/dodecyl glycol copolymer (Elfacos ST9) | 3.000 |
| stearoxytrimethylsilane | 1.000 |

13

| | |
|---|---|
| dimethicone (50 cstk) | 1.000 |
| Vitamin A alcohol (retinol) | 0.345 |
| methylparaben | 0.300 |
| fragrance | ·0.250 |
| propylparaben | 0.200 |
| Quaternium 15 (Dowicil 200) | 0.100 |
| disodium edetate | 0.100 |
| hydroquinone | 0.100 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of about one year. The stability is affected as a result of the somewhat limited water solubility of the hydroquinone.

EXAMPLE XI

A water-in-oil cream composition is prepared in accordance with the procedure of Example I with 0.5 g propyl gallate replacing the ascorbic acid and consists of the following ingredients:

| Ingredient | % w/w |
|---|---|
| mineral oil | 25.000 |
| hydroxyoctacosanyl hydroxystearate (Elfacos C26) | 5.000 |
| sorbitol solution | 5.000 |
| methoxy PEG-22/dodecyl glycol copolymer (Elfacos E200) | 5.000 |
| PEG-45/dodecyl glycol copolymer (Elfacos ST9) | 3.000 |

14

| | |
|---|---|
| stearoxytrimethylsilane | 1.000 |
| dimethicone (50 cstk) | 1.000 |
| Vitamin A alcohol (retinol) | 0.345 |
| methylparaben | 0.300 |
| fragrance | 0.250 |
| propylparaben | 0.200 |
| Quaternium 15 (Dowicil 200) | 0.100 |
| disodium edetate | 0.100 |
| propyl gallate | 0.050 |
| butylated hydroxytoluene | 0.050 |
| 50% aqueous NaOH | q.s. to pH 4.7 |
| distilled water | q.s. to 100.000 |

The resulting water-in-oil emulsion composition is an off-white, water-in-oil cream having a room temperature life stability of the retinol of about one year. The stability is affected as a result of the somewhat limited water solubility of the propyl gallate.

EXAMPLE XII

A water-in-oil cream composition is prepared in accordance with the procedure of Example I with 0.5 g nordihydroguiaretic acid replacing the ascorbic acid and consists of the following ingredients:

| Ingredient | % w/w |
|---|---|
| mineral oil | 25.000 |
| hydroxyoctacosanyl hydroxystearate (Elfacos C26) | 5.000 |
| sorbitol solution | 5.000 |
| methoxy PEG-22/dodecyl glycol copolymer (Elfacos E200) | 5.000 |

| | |
|---|---|
| PEG-45/dodecyl glycol copolymer (Elfacos ST9) | 3.000 |
| stearoxytrimethylsilane | 1.000 |
| dimethicone (50 cstk) | 1.000 |
| Vitamin A alcohol (retinol) | 0.345 |
| methylparaben | 0.300 |
| fragrance | 0.250 |
| propylparaben | 0.200 |
| Quaternium 15 (Dowicil 200) | 0.100 |
| disodium edetate | 0.100 |
| nordihydroguiaretic acid | 0.050 |
| butylated hydroxytoluene | 0.050 |
| 50%. aqueous NaOH | q.s. to pH 4.7 |
| deionized water | q.s. to 100.000 |

The resulting water-in-oil emulsion composition is an off-white cream having a room temperature shelf life stability of the retinol of just over one year. The stability is affected as a result of the somewhat limited water solubility of the nordihydroguiaretic acid.

EXAMPLE XIII

A water-in-oil cream composition is prepared in accordance with the procedure of Example I with 0.5.g monothioglycerol replacing the ascorbic acid and consists of the following ingredients:

| Ingredient | % w/w |
|---|---|
| mineral oil | 25.000 |
| hydroxyoctacosanyl hydroxystearate (Elfacos C26) | 5.000 |
| sorbitol solution | 5.000 |

| | |
|---|---|
| methoxy PEG-22/dodecyl glycol | 5.000 |
| copolymer (Elfacos E200) | |
| PEG-45/dodecyl glycol | 3.000 |
| copolymer (Elfacos ST9) | . |
| stearoxytrimethylsilane | 1.000 |
| dimethicone (50 cstk) | 1.000 |
| Vitamin A alcohol (retinol) | 0.345 |
| methylparaben | 0.300 |
| fragrance | 0.250 |
| propylparaben | 0.200 |
| Quaternium 15 (Dowicil 200) | 0.100 |
| disodium edetate | 0.100 |
| monothioglycerol | 0.050 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE XIV

A water-in-oil cream composition is prepared in accordance with the procedure of Example I with 0.5 g sodium thiosulphate replacing the ascorbic acid and consists of the following ingredients:

| Ingredient | % w/w |
|---|---|
| mineral oil | 25.000 |
| hydroxyoctacosanyl hydroxystearate | 5.000 |
| (Elfacos C26) | |
| sorbitol solution | 5.000 |

| | |
|---|---|
| methoxy PEG-22/dodecyl glycol copolymer (Elfacos E200) | 5.000 |
| PEG-45/dodecyl glycol copolymer (Elfacos ST9) | 3.000 |
| stearoxytrimethylsilane | 1.000 |
| dimethicone (50 cstk) | 1.000 |
| Vitamin A alcohol (retinol) | 0.345 |
| methylparaben | 0.300 |
| fragrance | 0.250 |
| propylparaben | 0.200 |
| Quaternium 15 (Dowicil 200) | 0.100 |
| disodium edetate | 0.100 |
| sodium thiosulphate | 0.050 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE XV

A water-in-oil cream composition is prepared in accordance with the procedure of Example I with 0.5 g sodium formaldehyde sulfoxylate replacing the ascorbic acid and consists of the following ingredients:

| Ingredient | % w/w |
|---|---|
| mineral oil | 25.000 |
| hydroxyoctacosanyl hydroxystearate (Elfacos C26) | 5.000 |
| sorbitol solution | 5.000 |

| | |
|---|---|
| methoxy PEG-22/dodecyl glycol copolymer (Elfacos E200) | 5.000 |
| PEG-45/dodecyl glycol copolymer (Elfacos ST9) | 3.000 |
| stearoxytrimethylsilane | 1.000 |
| dimethicone (50 cstk) | 1.000 |
| Vitamin A alcohol (retinol) | 0.345 |
| methylparaben | 0.300 |
| fragrance | 0.250 |
| propylparaben | 0.200 |
| Quaternium 15 (Dowicil 200) | 0.100 |
| disodium edetate | 0.100 |
| sodium formaldehyde sulfoxylate | 0.050 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE XVI

A water-in-oil cream composition is prepared in accordance with the procedure of Example I with 0.5 g 1,4 diazobicyclo-(2,2,2)-octane replacing the ascorbic acid and consists of the following ingredients:

| Ingredient | % w/w |
|---|---|
| mineral oil | 25.000 |
| hydroxyoctacosanyl hydroxystearate (Elfacos C26) | 5.000 |
| sorbitol solution | 5.000 |

| | |
|---|---|
| methoxy PEG-22/dodecyl glycol | 5.000 |
| copolymer (Elfacos E200) | |
| PEG-45/dodecyl glycol | 3.000 |
| copolymer (Elfacos ST9) | |
| stearoxytrimethylsilane | 1.000 |
| dimethicone (50 cstk) | 1.000 |
| Vitamin A alcohol (retinol) | 0.345 |
| methylparaben | 0.300 |
| fragrance | 0.250 |
| propylparaben | 0.200 |
| Quaternium 15 (Dowicil 200) | 0.100 |
| disodium edetate | 0.100 |
| 1,4-diazobicyclo-(2,2,2)-octane | 0.050 |
| butylated hydroxytoluene · | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE XVII

A water-in-oil cream composition is prepared generally in accordance with the procedure of Example I and consists of the following ingredients:

| Ingredient | % w/w |
|---|---|
| mineral oil | 5.700 |
| isopropyl palmitate | 5.000 |
| hydroxyoctacosanyl hydroxystearate | 2.800 |
| (Elfacos C26) | |
| sorbitol solution | 5.000 |

20

| | |
|---|---|
| methoxy PEG-22/dodecyl glycol | 2.300 |
| copolymer (Elfacos E200) | |
| PEG-45/dodecyl glycol | 1.400 |
| copolymer (Elfacos ST9) | |
| silicon dioxide | 1.000 |
| stearoxytrimethylsilane | 0.500 |
| dimethicone (50 cstk) | 1.000 |
| Vitamin A alcohol (retinol) | 0.345 |
| methylparaben | 0.300 |
| fragrance | 0.150 |
| propylparaben | 0.200 |
| phenoxyethanol | 1.000 |
| disodium edetate | 0.100 |
| ascorbic acid | 0.100 |
| butylated hydroxytoluene | 0.050 |
| 50% aqueous NaOH | q.s. to pH 4.7 |
| deionized water | q.s. to 100.000 |

The resulting water-in-oil emulsion composition is an off-white cream having a room temperature shelf life stability of the retinol of over two years.

EXAMPLE XVIII

A water-in-oil lotion composition is prepared according to the following procedure. In a suitably sized glass beaker are weighed 155.4 g C12-15 alcohols benzoate, 30.8 g cetearyl isononanoate, 4.9 g Polysorbate 21, 4.9 g Vitamin E acetate, 48.75 g oleth-3 phosphate, 20 g PEG-7 hydrogenated castor oil, 25 g talc, 7.5 g stearoxytrimethylsilane, and 30 g stearic acid. The mixture is heated to 80°C. In a separate glass beaker 620 g deionized water, 0.75 g Carbomer 934, 0.5 g disodium edetate, 7 g calcium hydroxide, 10 g glycerine and 1 g ascorbic acid are dispersed together and then heated to 80°C. and added to the first mixture with thorough agitation to form a water-in-oil emulsion. The emulsion is homogenized to refine the particle size and cooled to 50°C. and 1 g fragrance and 1 g dowicil 200 added. 3.45 g of retinol are added and purified water is added to return the batch weight to 1000 g. The emulsion is cooled to below 40°C. and filled into suitable packages. The resulting composition has the following formulation:

| Ingredient | % w/w |
|---|---|
| C12-15 alcohols benzoate | 15.540 |
| oleth-3 phosphate | 4.875 |
| cetearyl isononanoate | 3.080 |
| stearic acid | 3.000 |
| talc | 2.500 |
| PEG-7 hydrogenated castor oil | 2.000 |
| glycerine | 1.000 |
| stearoxytrimethylsilane | 0.750 |
| calcium hydroxide | 0.700 |
| Polysorbate 21 | 0.490 |
| Vitamin E acetate | 0.490 |
| Vitamin A alcohol (retinol) | 0.345 |
| fragrance | 0.100 |
| Quaternium 15 (Dowicil 200) | 0.100 |
| ascorbic acid | 0.100 |
| disodium edetate | 0.050 |
| Carbomer 934 | 0.075 |
| deionized water | q.s. to 100.000 |

The resulting water-in-oil emulsion composition is an off-white lotion and has good shelf life stability.

EXAMPLE XIX (comparative example)

A water-in-oil lotion composition is prepared according to the following procedure. In a suitably sized glass beaker are weighed 84.7 g C12-15 alcohols benzoate, 15 g cetearyl isononanoate, 2.45 g Polysorbate 21, 25 g PEG-7 hydrogenated castor oil, 1.54 g dimethicone, 10 g Arlacel 481, 10 g PVP/eicosene copolymer, 20 g silicon dioxide, and 5 g phenoxyethanol and the mixture is heated to 80°C. In a separate glass beaker 320 g deionized water, 0.25 g disodium edetate, and 0.5 g ascorbic acid are dispersed together and then heated to 80°C. and added to the first mixture with thorough agitation to form a water-in-oil emulsion. The emulsion is homogenized to refine the particle size and is cooled to 50°C. and 0.5 g fragrance added. 1.725 g of retinol are added and deionized water is added to return the batch weight to 500 g. The emulsion is cooled to below 40°C. and filled into suitable packages. The resulting composition has the following formulation:

| Ingredient | % w/w |
|---|---|
| C12-15 alcohols benzoate | 16.340 |
| PEG-7 hydrogenated castor oil | 5.000 |
| silicon dioxide | 4.000 |
| cetearyl isononanoate | 3.000 |
| Arlacel 481 | 2.000 |
| PVP/eicosene copolymer | 2.000 |
| phenoxyethanol | 1.000 |
| Polysorbate 21 | 0.490 |
| Vitamin A alcohol (retinol) | 0.345 |
| dimethicone | 0.308 |
| fragrance | 0.100 |
| | |
| ascorbic acid | 0.100 |
| disodium edetate | 0.050 |
| deionized water | q.s. to 100.000 |

The resulting water-in-oil emulsion composition is a smooth off-white cream and three month retinol room temperature stability of this formulation compares favorably with other compositions whose retinol shelf life stability is two years or more.

EXAMPLE XX

A water-in-oil cream composition is prepared according to the following procedure. In a suitable sized glass beaker 250 g mineral oil, 2 g propylparaben, 60 g Elfacos C-26, 50 g Elfacos E200, 30 g Elfacos ST-9, 10 g stearoxytrimethylsilane, 10 g dimethicone 50cstk, and 0.5 g butylated hydroxytoluene (BHT) are heated to 80°C. and mixed. In a separate glass beaker approximately 520 g purified water USP, 50 g sorbitol solution 70%, 3 g methylparaben, 1 g disodium edetate and 1 g ascorbic acid are mixed, adjusted to pH 4.7 with dilute sodium hydroxide q.s., heated to 80°C. and then added to the first mixture with agitation to form a water-in-oil emulsion. The emulsion is homogenized to further refine the particle size and cooled to about 50°C. with ordinary mixing. 1 g Dowicil 200 and 2.5 g fragrance are added and then 5.5 g of retinyl palmitate are added. Deionized water is added to return the batch weight to 1000 g and the batch is mixed until uniform and the temperature is below 40°C. The finished batch is filled into suitable containers and has the following formulation:

| Ingredient | % w/w |
|---|---|
| mineral oil | 25.000 |
| hydroxyoctacosanyl hydroxystearate (Elfacos C26) | 6.000 |
| sorbitol solution | 5.000 |
| methoxy PEG-22/dodecyl glycol copolymer (Elfacos E200) | 5.000 |
| PEG-45/dodecyl glycol copolymer (Elfacos ST9) | 3.000 |
| stearoxytrimethylsilane | 1.000 |
| dimethicone (50 cstk) | 1.000 |
| retinyl palmitate | 0.550 |
| methylparaben | 0.300 |
| fragrance | 0.250 |
| propylparaben | 0.200 |
| Quaternium 15 (Dowicil 200) | 0.100 |
| disodium edetate | 0.100 |
| ascorbic acid | 0.100 |
| butylated hydroxytoluene | 0.050 |
| 50% aqueous NaOH | q.s. to pH 4.7 |
| deionized water | q.s. to 100.000 |

The resulting water-in-oil emulsion composition is a smooth off-white cream and has good shelf life stability.

EXAMPLE XXI

A water-in-oil cream composition is prepared in accordance with the procedure of Example XX and has the following ingredients:

| Ingredient | % w/w |
|---|---|
| mineral oil | 25.000 |
| hydroxyoctacosanyl hydroxystearate (Elfacos C26) | 6.000 |
| sorbitol solution | 5.000 |
| methoxy PEG-22/dodecyl glycol copolymer (Elfacos E200) | 5.000 |
| PEG-45/dodecyl glycol copolymer (Elfacos ST9) | 3.000 |
| stearoxytrimethylsilane | 1.000 |
| dimethicone (50 cstk) | 1.000 |
| retinyl acetate | 0.340 |
| methylparaben | 0.300 |
| fragrance | 0.250 |
| propylparaben | 0.200 |
| Quaternium 15 (Dowicil 200) | 0.100 |
| disodium edetate | 0.100 |
| ascorbic acid | 0.100 |
| butylated hydroxytoluene | 0.050 |
| 50% aqueous NaOH | q.s. to pH 4.7 |
| deionized water | q.s. to 100.000 |

The resulting water-in-oil emulsion composition is a smooth off-white cream and has good shelf life stability.

**Claims**

1. A skin care composition consisting of a water-in-oil emulsion base comprising:
   a) an antioxidant system comprising at least one water-soluble antioxidant and at least one oil-soluble antioxidant;
   b) a chelating agent; and
   c) at least one retinoid selected from Vitamin A alcohol, Vitamin A aldehyde or a Vitamin A ester.

2. The skin care composition of Claim 1, wherein the retinoid is Vitamin A alcohol.

3. The skin care composition of claim 1 or claim 2, wherein the antioxidant system contains at least one antioxidant selected from ascorbic acid, sodium sulfite, sodium metabisulfite, sodium bisulfite, sodium thiosulfite, sodium formaldehyde sulfoxylate, isoascorbic acid, thioglycerol, thiosorbitol, thiourea, thioglycolic acid, cysteine hydrochloride, 1,4-diazobicyclo-(2,2,2)-octane or a mixture thereof.

4. The skin care composition of claim 3, wherein the antioxidant system contains ascorbic acid.

5. The skin care composition of any one of claims 1 to 4, wherein the antioxidant system contains at least one antioxidant selected from butylated hydroxytoluene (BHT), ascorbyl palmitate, butylated hydroxyanisole, $\alpha$-tocopherol, phenyl-$\alpha$-naphthylamine or a mixture thereof.

25

6. The skin care composition of claim 5, wherein the antioxidant system contains butylated hydroxytoluene.

7. The skin care composition of any one of claims 1 to 6, wherein the antioxidant system contains at least one antioxidant selected from hydroquinone, propyl gallate, nordihydroguiaretic acid or a mixture thereof.

8. The skin care composition of any one of claims 1 to 7, wherein the chelating agent is selected from ethylenediamine tetracetic acid (EDTA) and derivatives and salts thereof, dihydroxyethyl glycine, citric acid, tartaric acid or a mixutre thereof.

9. The skin care composition of claim 8, wherein the chelating agent is ethylenediamine tetracetic acid or a derivative or salt thereof.

10. A method for producing a skin care composition according to any one of claims 1 to 9, which comprises: mixing together the components which will form the oil phase; mixing together the components which will form the water phase; and adding the water phase to the oil phase, wherein the retinoid is optionally added after the addition of the water phase to the oil phase.

## Patentansprüche

1. Hautpflegezusammensetzung, bestehend aus einer Wasserin-Öl-Emulsionsbasis, umfassend:
   a) ein Antioxidationsmittelsystem, das mindestens ein wasserlösliches Antioxidans und mindestens ein öllösliches Antioxidans umfaßt,
   b) ein ein Chelat bildendes Mittel, und
   c) mindestens ein Retinoid ausgewählt aus Vitamin A-Alkohol, Vitamin A-Aldehyd oder einem Vitamin A-Ester.

2. Hautpflegezusammensetzung nach Anspruch 1, in der das Retinoid Vitamin A-Alkohol ist.

3. Hautpflegezusammensetzung nach Anspruch 1 oder Anspruch 2, in der das Antioxidationsmittelsystem mindestens ein Antioxidans ausgewählt aus Ascorbinsäure, Natriumsulfit, Natriummetabisulfit, Natriumbisulfit, Natriumthiosulfit, Natriumformaldehydsulfoxylat, Isoascorbinsäure, Thioglycerin, Thiosorbit, Thioharnstoff, Thioglycolsäure, Cystein-Hydrochlorid, 1,4-Diazobicyclo-(2,2,2)-oktan oder ein Gemisch davon enthält.

4. Hautpflegezusammensetzung nach Anspruch 3, in der das Antioxidationsmittelsystem Ascorbinsäure enthält.

5. Hautpflegezusammensetzung nach einem der Ansprüche 1 bis 4, in der das Antioxidationsmittelsystem mindestens ein Antioxidans ausgewählt aus butyliertem Hydroxytoluol (BHT), Ascorbylpalmitat, butyliertem Hydroxyanisol, α-Tocopherol, Phenyl-α-naphthylamin oder einem Gemisch davon enthält.

6. Hautpflegezusammensetzung nach Anspruch 5, in der das Antioxidationsmittelsystem butyliertes Hydroxytoluol enthält.

7. Hautpflegezusammensetzung nach einem der Ansprüche 1 bis 6, in der das Antioxidationsmittelsystem mindestens ein Antioxidans ausgewählt aus Hydrochinon, Propylgallat, Nordihydroguajaretsäure oder ein Gemisch davon enthält.

8. Hautpflegezusammensetzung nach einem der Ansprüche 1 bis 7, in der das chelatbildende Mittel ausgewählt ist aus Ethylendiamintetraessigsäure (EDTA) und Derivaten und Salzen davon, Dihydroxyethylglycin, Zitronensäure, Weinsäure oder einem Gemisch davon.

9. Hautpflegezusammensetzung nach Anspruch 8, in der das chelatbildende Mittel Ethylendiamintetraessigsäure oder ein Derivat oder Salz davon ist.

10. Verfahren zur Herstellung einer Hautpflegezusammensetzung nach einem der Ansprüche 1 bis 9, das umfaßt: das Zusammenmischen der Komponenten, die die Ölphase bilden, das Zusammenmischen der Komponenten, die die Wasserphase bilden und das Zugeben der Wasserphase zu der Ölphase, wobei das Retinoid wahlweise nach der Zugabe der Wasserphase zu der Ölphase zugegeben wird.

EP 0 440 398 B1

**Revendications**

1. Une composition pour les soins de la peau, constituée d'une base d'émulsion eau-dans-huile comportant :
   a) un système antioxydant comportant au moins un oxydant soluble dans l'eau et au moins un antioxydant soluble dans l'huile ;
   b) un agent chelatant ; et
   c) au moins un rétinoïde choisi parmi l'alcool de vitamine A, l'aldéhyde de vitamine A ou un ester de vitamine A.

2. La composition pour les soins de la peau de la revendication 1, dans laquelle le rétinoïde est un alcool de vitamine A.

3. La composition pour les soins de la peau de la revendication 1 ou de la revendication 2, dans laquelle le système antioxydant renferme au moins un antioxydant choisi parmi l'acide ascorbique, le sulfite de sodium, le métabisulfite de sodium, le bisulfite de sodium, le thiosulfite de sodium, le formaldéhyde sulfoxylate de sodium, l'acide iso-ascorbique, le thioglycérol, le thiosorbitol, la thiourée, l'acide thioglycolique, le chlorhydrate de cystéine, le 1,4-diazobicylo-(2,2,2)-octane ou un mélange de ceux-ci.

4. La composition pour les soins de la peau de la revendication 3, dans laquelle le système antioxydant contient de l'acide ascorbique.

5. La composition pour les soins de la peau de l'une quelconque des revendications 1 à 4, dans laquelle le système antioxydant renferme au moins un antioxydant choisi parmi l'hydroxytoluène butylé (BHT), le palmitate d'ascorbyle, l'hydroxy-anisole butylé, l'$\alpha$-tocophérol, la phényl-$\alpha$-naphtylamine ou un mélange de ceux-ci.

6. La composition pour les soins de la peau selon la revendication 5, dans laquelle le système antioxydant renferme un hydroxytoluène butylé.

7. La composition pour les soins de la peau de l'une quelconque des revendications 1 à 6, dans laquelle le système antioxydant renferme au moins un antioxydant choisi parmi l'hydroquinone, le gallate de propyle, l'acide nordihydroguiarétique ou un mélange de ceux-ci.

8. La composition pour les soins de la peau de l'une quelconque des revendications 1 à 7, dans laquelle l'agent chélatant est choisi parmi l'acide éthylènediamine tétracétique (EDTA) ainsi que ses dérivés et ses sels, la dihydroxyéthyl glycine, l'acide citrique, l'acide tartarique ou un mélange de ceux-ci.

9. La composition pour les soins de la peau de la revendication 8, dans laquelle l'agent chélatant est l'acide éthylènediamine tétracétique ou un dérivé ou un sel de celui-ci.

10. Un procédé pour la préparation d'une composition pour les soins de la peau selon l'une quelconque des revendications 1 à 9, procédé selon lequel : on mélange ensemble les composés qui formeront la phase huileuse ; on mélange ensemble les composés qui formeront la phase aqueuse ; et on ajoute la phase aqueuse à la phase huileuse, le rétinoïde étant facultativement ajouté après l'addition de la phase aqueuse à la phase huileuse.

27